# EUROPÄISCHE PATENTSCHRIFT

(11) **EP 3 013 289 B1**
(45) Veröffentlichungstag und Bekanntmachung des Hinweises auf die Patenterteilung: **22.03.2017**
(21) Anmeldenummer: 15759368.2
(22) Anmeldetag: 21.07.2015
(51) Int. Cl.: A61F 9/007, A61B 17/32

(54) **VORRICHTUNG ZUM SCHNEIDEN VON GEWEBE**
DEVICE FOR CUTTING TISSUE
DISPOSITIF DE COUPE DE TISSUS

(30) Priorität: 26.09.2014 DE 102014219616
(43) Veröffentlichungstag der Anmeldung: 04.05.2016
(73) Patentinhaber: Geuder AG, 69126 Heidelberg (DE)
(72) Erfinder: GEUDER, Volker, 69126 Heidelberg (DE)
(74) Vertreter: Patent- und Rechtsanwälte Ullrich & Naumann
(86) Internationale Anmeldenummer: PCT/DE2015/200410
(87) Internationale Veröffentlichungsnummer: WO 2016/045670

(56) Entgegenhaltungen:
- DE-A1-102010 050 337
- US-A- 4 811 734
- US-A- 4 834 729
- US-A1- 2010 106 054
- US-A1- 2011 313 346

## Beschreibung

Die Erfindung betrifft eine Vorrichtung zum Schneiden und Absaugen von Gewebe aus dem menschlichen oder tierischen Körper, vorzugsweise zum Einsatz in der Ophthalmologie, insbesondere zur Vitrektomie, zum Netzhautpeeling etc., mit einer äußeren Röhre und einer in der äußeren Röhre konzentrisch mit geringem Spiel alternierend verschiebbaren inneren Röhre, wobei die äußere Röhre am freien Ende geschlossen ist und nahe dem freien Ende eine erste und eine zweite seitliche Öffnung mit jeweils zumindest einer inneren Schneidkante aufweist, wobei die innere Röhre am freien Ende offen ist und dort eine äußere Schneidkante aufweist, wobei die innere Röhre nahe dem freien Ende mindestens eine seitliche Öffnung mit mindestens einer weiteren äußeren Schneidkante aufweist und wobei die Schneidkanten der äußeren Röhre und der inneren Röhre beim Verschieben der inneren Röhre schneidend zusammen wirken.

Grundsätzlich geht es hier um ein chirurgisches Schneidinstrument zur Entnahme von Gewebe. Mit dem Instrument lässt sich das Gewebe - am bzw. im Körper - schneiden und vom Körper bzw. aus dem Körper heraus absaugen. Im Konkreten kann es sich dabei um ein ganz besonderes Schneidinstrument handeln, mit dem im Rahmen der Vitrektomie der Glaskörper im Auge zerstört bzw. zerkleinert und aus dem Auge entfernt/abgesaugt wird. Auch lassen sich mit diesem Instrument Blut, Blutgerinnsel und bindegewebsähnliche Veränderungen sowie Bereiche der Netzhaut im Rahmen eines Netzhautpeelings entfernen. Eine Vorrichtung der gattungsgemäßen Art eignet sich grundsätzlich zum Einsatz in der Augenchirurgie.

Zum Stand der Technik sei auf die DE 10 2010 050 337 A1 verwiesen. Aus dieser Druckschrift ist eine gattungsgemäße Vorrichtung bekannt, wobei dort sowohl die äußere Röhre als auch die innere Röhre jeweils zwei seitliche Ausnehmungen mit Doppelschneidfunktion aufweisen. Ähnliche Vorrichtungen sind aus der DE 10 2013 201 784 A1, US 5,630,827, US 5,474,532 und US 5,106,364 bekannt. Des Weiteren zeigen die Dokumente US 2010/106054 A1, US 2011/313346 A1 und US 4,834,729 A entsprechende Vorrichtungen.

Bei der gattungsbildenden Vorrichtung nach der DE 10 2010 050 337 A1 sind in der äußeren Röhre zwei parallel zueinander ausgebildete Schlitze mit parallelen Schneidkanten vorgesehen. Des Weiteren ist das freie Ende der inneren Röhre offen und nahe dem freien Ende eine seitliche Öffnung mit einer äußeren Schneidkante angeordnet.

Bei der gattungsbildenden Vorrichtung ist problematisch, dass je nach zu schneidendem Gewebe eine recht hohe Schneidkraft aufgebracht werden muss, um das Gewebe zu zerteilen. Des Weiteren ist problematisch, dass das Gewebe nicht mit einem sauberen Schnitt abgetrennt, sondern vielmehr durch die Schneidkanten ausgestanzt wird. Insbesondere bei einer Verwendung der Vorrichtung in der Ophthalmologie muss das Gewebe im Augeninneren mit einem möglichst glatten Schnitt abgetrennt werden, um das empfindliche Auge nicht zu schädigen. Ein Ausstanzen von Gewebe kann am Auge zu erheblichen Irritationen und Wundheilungsstörungen führen und ist somit zu vermeiden.

Des Weiteren ist problematisch, dass bei der gattungsbildenden Vorrichtung während des Bewegungsablaufs der inneren Röhre eine konstante Strömung von Aspirationsflüssigkeit und abgetrenntem Gewebe nicht erreichbar ist. Daher entsteht im Körper bzw. Auge ein - wenn auch geringer - alternierender Unterdruck, der ebenfalls zu Irritationen am Auge führt, was sich negativ auf den Heilungsprozess auswirkt.

Der vorliegenden Erfindung liegt daher die Aufgabe zugrunde, eine Vorrichtung zum Schneiden und Absaugen von Gewebe aus dem menschlichen oder tierischen Körper derart auszugestalten und weiterzubilden, dass mit konstruktiv einfachen Mitteln ein sauberes Schneiden von Gewebe möglich ist und Irritationen am Gewebe sowie Druckschwankungen im Körper minimiert sind.

Erfindungsgemäß wird die voranstehende Aufgabe durch die Merkmale des Anspruchs 1 gelöst. Danach ist die in Rede stehende Vorrichtung dadurch gekennzeichnet, dass die erste und die zweite Öffnung jeweils als asymmetrischer Kreis, als asymmetrische Ellipse oder als asymmetrisches Oval ausgebildet sind und dass durch den asymmetrischen Bereich der ersten und zweiten Öffnung an der äußeren Röhre ein dünner Steg ausgebildet ist.

In erfindungsgemäßer Weise ist zunächst erkannt worden, dass durch die Anordnung von zwei als asymmetrischer Kreis, Ellipse oder Oval ausgebildeten Öffnungen die Schneidleistung der Vorrichtung in überraschender Weise verbessert ist. Durch den kreisförmigen, elliptischen bzw. ovalen Bereich ist die Länge der äußeren Schneidkanten maximiert und es wird eine punktuelle Schneidkraft erreicht. Dadurch wird eine glatte Schnittführung erzielt und ein Ausstanzen des zu schneidenden Gewebes vermieden. Des Weiteren wird durch diesen Bereich der Öffnungen ein ideales Strömungsverhalten unter Minimierung von Verwirbelungen der Aspirationsflüssigkeit erreicht, so dass bei der Aspiration eine konstante Strömung durch die innere Röhre gewährleistet ist.

In weiter erfindungsgemäßer Weise ist erkannt worden, dass durch die asymmetrische Ausgestaltung der Öffnungen ein dünner Steg in der äußeren Röhre realisierbar ist, so dass weitere Schneidkanten vorgesehen sein können, wobei sich diese Ausgestaltung nicht in negativer Weise auf das Strömungsverhalten der Aspirationsflüssigkeit auswirkt.

Um eine ideale Schneidleistung bei möglichst geringer Schneidkraft zu erreichen, kann der Steg zumindest abschnittsweise orthogonal zur Längsachse der äußeren Röhre verlaufen.

Zur Realisierung einer möglichst einfachen Konstruktion und Optimierung des Strömungsverhaltens können die erste Öffnung und die zweite Öffnung der äußeren Röhre achsensymmetrisch zueinander ausgebildet sein, wobei der Steg zumindest abschnittsweise einen Teil einer Symmetrieachse bildet. Mit anderen Worten kann der Steg zumindest abschnittsweise auf der zwischen der ersten Öffnung und der zweiten Öffnung verlaufenden Spiegelachse liegen. Ein weiterer Vorteil dieser Ausgestaltung liegt darin, dass durch die achsensymmetrische Anordnung auf besonders einfache Weise erreichbar ist, dass zu jedem Zeitpunkt des Bewegungsablaufs der inneren Röhre der gleiche Strömungsquerschnitt in die innere Röhre hinein besteht.

In einer vorteilhaften Ausgestaltung können an der ersten Öffnung und an der zweiten Öffnung jeweils eine erste und eine zweite Schneidkante ausgebildet sein. Somit sind in der äußeren Röhre insgesamt vier innere Schneidkanten angeordnet, so dass die Schneidleistung der Vorrichtung mit konstruktiv einfachen Mitteln erhöht ist.

In vorteilhafter Weise ist die erste Schneidkante in dem kreisförmigen, elliptischen oder ovalen Bereich der ersten und der zweiten Öffnung ausgebildet. Alternativ oder zusätzlich kann die zweite Schneidkante in dem asymmetrischen Bereich der ersten und der zweiten Öffnung ausgebildet sein.

Wie bereits zuvor erwähnt, verfügt die äußere Röhre über innere Schneidkanten und die innere Röhre über äußere Schneidkanten, wobei die jeweiligen Schneidkanten bei der Hubbewegung der inneren Röhre aneinander vorbei gleiten, wodurch sich sowohl beim Vorwärtshub als auch beim Rückwärtshub die Schneidwirkung ergibt. Von den Schneidkanten der äußeren Röhre aus können sich Schneidflächen nach außen erstrecken, die durch die Materialstärke der äußeren Röhre definiert sind. Dabei ist denkbar, dass sich die Schneidfläche bzw. die Schneidflächen orthogonal zu der Längsachse der äußeren Röhre erstreckt bzw. erstrecken. Des Weiteren können sich die Schneidflächen bzw. die Schneidfläche unter einem Winkel zu der Längsachse der äußeren Röhre erstrecken, d.h. die Schneidfläche bzw. Schneidflächen verlaufen schräg zu der Längsachse der äußeren Röhre. Je nach dem, was für ein Gewebe geschnitten werden soll, kann die Schneidfläche entsprechend angepasst sein. Dabei ist des Weiteren denkbar, dass bspw. die an dem Steg angeordneten Schneidkanten der ersten und zweiten Öffnung orthogonal zu der Längsachse der äußeren Röhre ausgebildet sind und die von der ersten Schneidkante nach außen verlaufende Schneidfläche der ersten Öffnung sowie der zweiten Öffnung schräg zu der Längsachse der äußeren Röhre angeordnet sind.

Um die Vorrichtung in idealer Weise an das zu schneidende Gewebe anzupassen, können die Schneidfläche bzw. die Schneidflächen linear und/oder konkav ausgebildet sein. Bspw. können die an dem Steg angeordneten Schneidflächen linear ausgebildet sein, wohingegen die an der ersten Schneidkante der ersten und zweiten Öffnung angeordneten Schneidflächen konkav ausgebildet sind.

In weiter vorteilhafter Weise sind die von der ersten Schneidkante nach außen verlaufende erste Schneidfläche der ersten Öffnung und die von der ersten Schneidkante nach außen verlaufende erste Schneidfläche der zweiten Öffnung V-förmig zueinander angeordnet. D.h., dass die ersten Schneidflächen der ersten Öffnung und der zweiten Öffnung jeweils schräg zu der Längsachse der äußeren Röhre verlaufen können, wobei die Schneidflächen in besonders vorteilhafter Weise in entgegengesetzten Winkeln zu der Längsachse angeordnet sind. Somit kann die erste Schneidfläche bei einer Vorwärtsbewegung der inneren Röhre im gleichen Winkel zur inneren Röhre stehen wie die zweite Schneidfläche bei einer Rückwärtsbewegung der inneren Röhre.

Zur weiteren Maximierung der Schneidleistung kann die innere Röhre nahe dem freien Ende zwei benachbarte seitliche Öffnungen aufweisen, die insgesamt mit drei oder vier äußeren Schneidkanten ausgestattet sind.

Des Weiteren ist denkbar, dass die äußere Röhre eine dritte und eine vierte seitliche Öffnung aufweist, die analog zur ersten und zweiten Öffnung ausgebildet sind. Die dritte und vierte seitliche Öffnung kann dabei axial oder in Umfangsrichtung beabstandet zu der ersten und zweiten Öffnung angeordnet sein, wobei die dritte und vierte Öffnung jeweils als asymmetrischer Kreis, als asymmetrische Ellipse oder als asymmetrisches Oval ausgebildet sein können und durch den asymmetrischen Bereich der dritten und vierten Öffnung an der äußeren Röhre ein weiterer dünner Steg ausgebildet sein kann.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1 bis Fig. 3: in geschnittenen Ansichten, teilweise, ein Ausführungsbeispiel einer erfindungsgemäßen Vorrichtung zum Schneiden und Absaugen von Gewebe, wobei die Figuren den Bewegungsablauf der inneren Röhre in der äußeren Röhre und die dort jeweils vorgesehenen Öffnungen und Schneidkanten zeigen,
- Fig. 4 bis Fig. 6: in schematischen Ansichten, teilweise, das Ausführungsbeispiel gemäß Fig. 1 bis 3, wobei die Figuren den Bewegungsablauf der inneren Röhre in der äußeren Röhre und die dort jeweils vorgesehenen Öffnungen und Schneidkanten zeigen und
- Fig. 7 bis Fig. 9: in schematischen Aufsichten, teilweise, das Ausführungsbeispiel gemäß Fig. 1 bis 3, wobei die Figuren den Bewegungsablauf der inneren Röhre in der äußeren Röhre und die dort jeweils vorgesehenen Öffnungen und Schneidkanten zeigen.

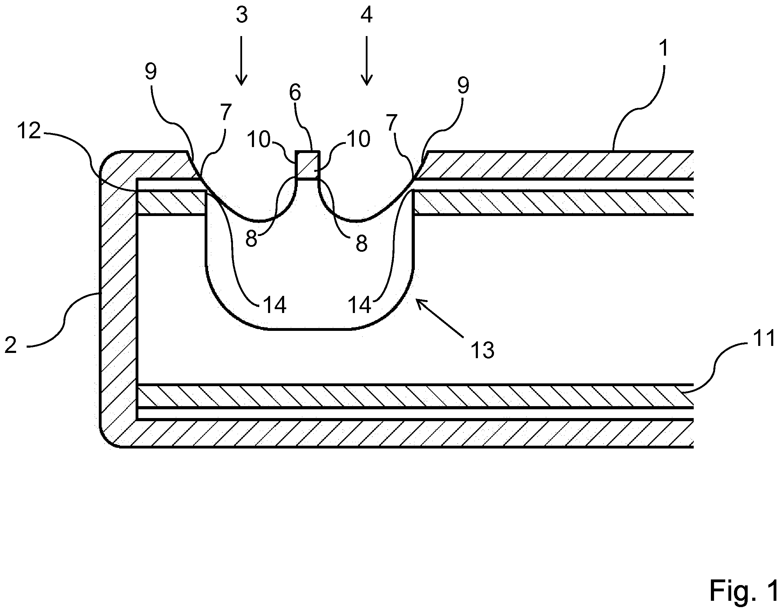

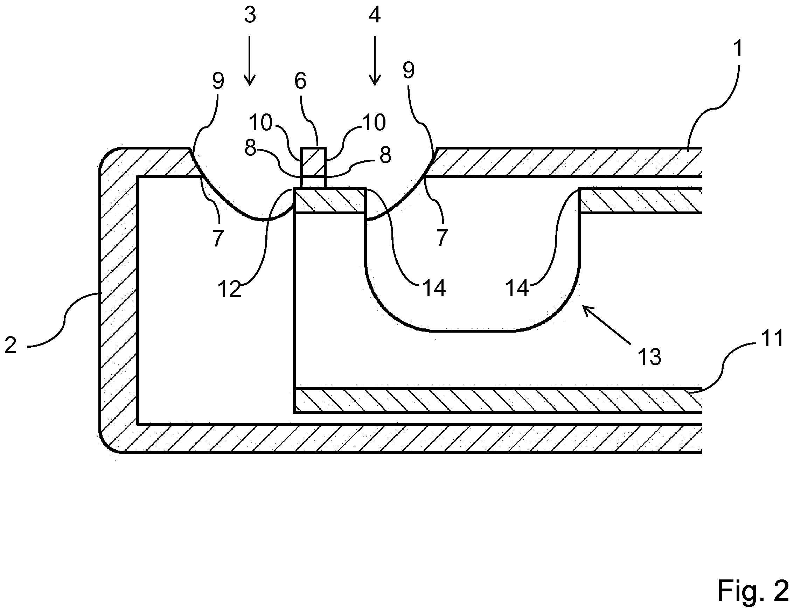

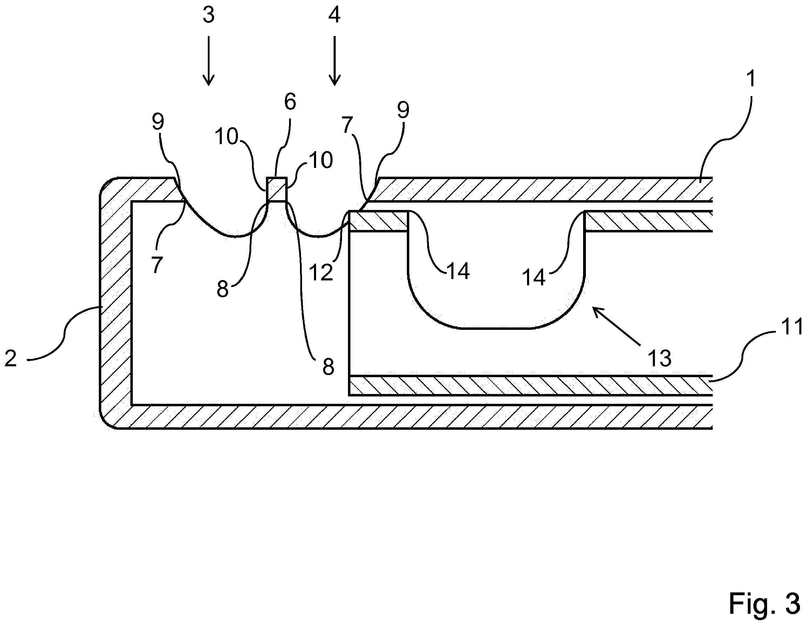

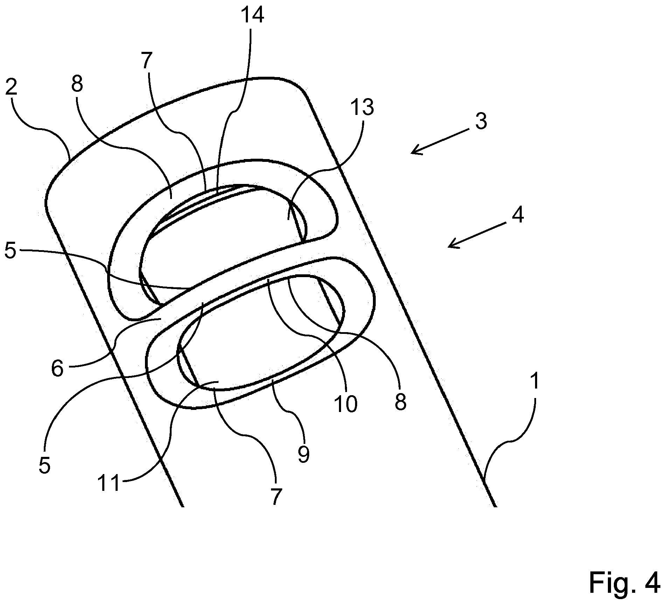

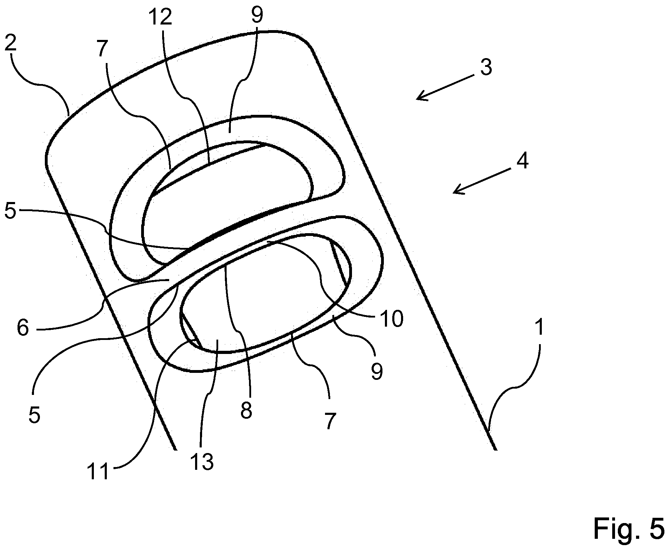

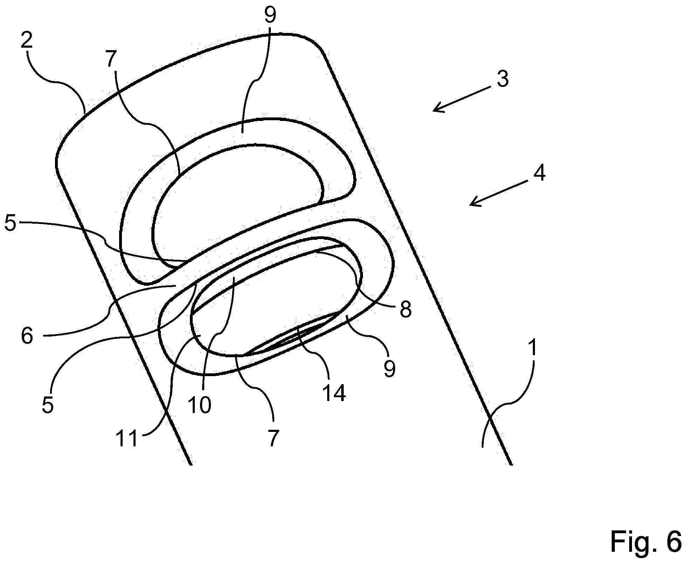

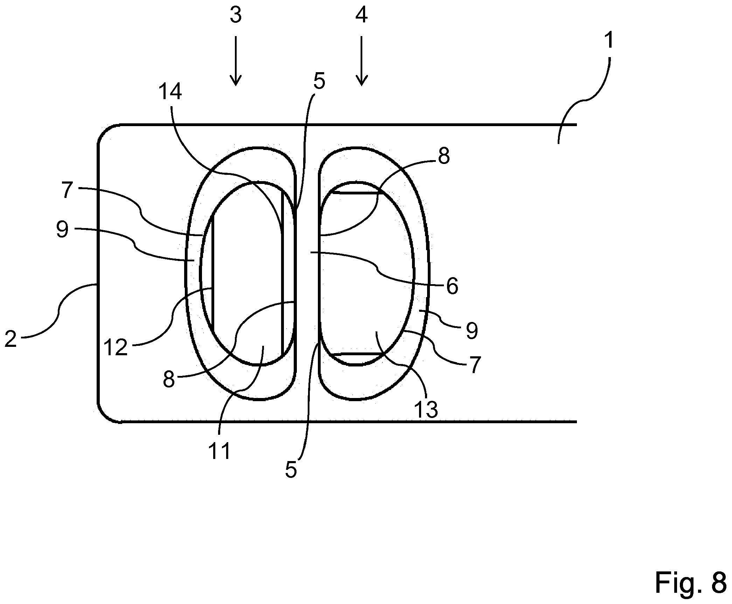

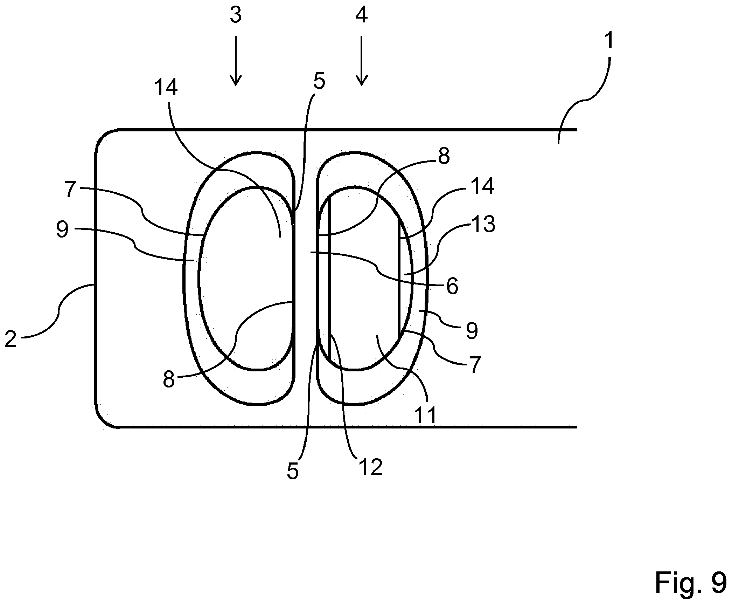

Die Figuren 1 bis 9 zeigen in schematischen Ansichten, stark vergrößert, den Arbeitsbereich der erfindungsgemäßen Vorrichtung, wobei es sich hier um einen sog. Vitrektor zum Einsatz in der Vitrektomie handelt, nämlich um eine Vorrichtung zur Glaskörperentfernung aus dem menschlichen Auge.

Die Figuren 1 bis 9 zeigen gemeinsam den Bewegungsablauf und dabei die jeweilige Stellung der beweglichen Teile zueinander.

Im Konkreten zeigen die Figuren 1 bis 9 - teilweise - die äußere Röhre 1, die am freien Ende 2 geschlossen ist. Bei dem in den Figuren 1 bis 9 gezeigten Ausführungsbeispiel weist die äußere Röhre eine erste Öffnung 3 und eine zweite Öffnung 4 auf. Die erste Öffnung 3 und die zweite Öffnung 4 sind jeweils als asymmetrische Ellipse bzw. als asymmetrisches Oval ausgebildet. Dabei ist deutlich zu erkennen, dass der asymmetrische Bereich 5 der ersten Öffnung 3 und der zweiten Öffnung 4 an der äußeren Röhre 1 einen dünnen Steg 6 definieren.

Des Weiteren sind an der ersten Öffnung 3 und der zweiten Öffnung 4 jeweils eine erste Schneidkante 7 und eine zweite Schneidkante 8 ausgebildet. Die äußere Röhre 1 verfügt somit über insgesamt vier Schneidkanten 7, 8.

Von den ersten Schneidkanten 7 erstrecken sich erste Schneidflächen 9 nach außen, die durch die Materialstärke der äußeren Röhre 1 definiert sind. Die ersten Schneidflächen 9 verlaufen schräg zu der Längsachse der äußeren Röhre 1 und sind V-förmig zueinander angeordnet. Des Weiteren ist deutlich zu erkennen, dass die ersten Schneidflächen 9 konkav ausgebildet sind.

Von den zweiten Schneidkanten 8 erstrecken sich weitere zweite Schneidflächen 10 nach außen, die ebenfalls durch die Materialstärke der äußeren Röhre 1 definiert sind. Im Gegensatz zu den ersten Schneidflächen 9 erstrecken sich die zweiten Schneidflächen 10 orthogonal zu der Längsachse der äußeren Röhre 1 und sind linear ausgebildet.

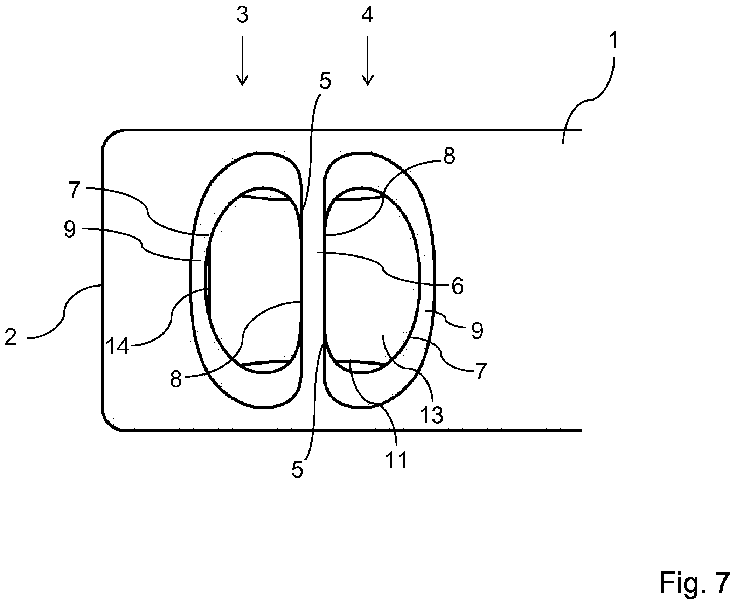

Insbesondere in den Figuren 7 bis 9 ist deutlich gezeigt, dass die erste Öffnung 3 und die zweite Öffnung 4 achsensymmetrisch zueinander ausgebildet sind, wobei der Steg 6 in der Symmetrieachse der ersten Öffnung 3 und der zweiten Öffnung 4 liegt.

In der äußeren Röhre 1 ist konzentrisch, mit geringem Spiel alternierend verschiebbar, eine innere Röhre 11 angeordnet. Die Figuren 1 bis 9 lassen erkennen, dass die innere Röhre 11 an ihrem vorderen freien Ende offen ist. Entsprechend ist dort eine erste Schneidkante 12 ausgebildet. Je nach Auslegung des Hubs der inneren Röhre 11 schneidet die erste Schneidkante 12 jedes mal dann, wenn sie an einer gegen die Bewegungsrichtung der inneren Röhre 11 gerichteten Schneidkante 7, 8 der äußeren Röhre 1 vorbeigleitet. Bei dem in den Figuren 1 bis 9 gezeigten Ausführungsbeispiel schneidet daher die erste Schneidkante 12 zweimal, wenn sie nämlich bei entsprechend ausgelegtem Hub zweimal an einer jeweils entgegengerichteten Schneidkante 7, 8 der äußeren Röhre 1 vorbeigeführt wird.

Nun kann die innere Röhre 11 mit einer oder mehreren Öffnungen 13 ausgestattet sein. Im hier dargestellten Ausführungsbeispiel weist die innere Röhre 11 eine Öffnung 13 auf, die zwei zusätzliche äußere Schneidkanten 14 aufweist, die beim Vorbeigleiten an entgegengesetzt gerichteten Schneidkanten 7, 8 der äußeren Röhre 1 einen Schneidvorgang bewirken.

Sowohl bei Vorkehrung einer einzigen Öffnung 13 in der inneren Röhre 11 als auch bei Vorkehrung von mehreren Öffnungen 13 in der inneren Röhre 11 können diese derart dimensioniert und positioniert sein, dass bei geeigneter Bewegung der inneren Röhre 11 innerhalb der äußeren Röhre 1 stets der gleiche Strömungsquerschnitt wirkt, nämlich dann, wenn die eine Öffnung 3, 4 in der äußeren Röhre 1 geöffnet oder geschlossen wird und die andere Öffnung 3, 4 in der äußeren Röhre 1 geschlossen oder geöffnet wird. In einer Mittelposition werden somit die Öffnungen 3, 4 in der äußeren Röhre 1 jeweils hälftig durch die Wandung der inneren Röhre 11 geschlossen bzw. entsprechend der Position der Schneidkante 14 der inneren Röhre 11 geöffnet sein.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass das voranstehend beschriebene Ausführungsbeispiel der erfindungsgemäßen Vorrichtung lediglich zur Erörterung der beanspruchten Lehre dient, diese jedoch nicht auf das Ausführungsbeispiel einschränkt.

### Bezugszeichenliste

- 1: äußere Röhre
- 2: freies Ende (äußere Röhre)
- 3: erste Öffnung (äußere Röhre)
- 4: zweite Öffnung (äußere Röhre)
- 5: asymmetrischer Bereich
- 6: Steg
- 7: erste Schneidkante (äußere Röhre)
- 8: zweite Schneidkante (äußere Röhre)
- 9: erste Schneidfläche
- 10: zweite Schneidfläche
- 11: innere Röhre
- 12: erste Schneidkante (innere Röhre)
- 13: Öffnung (innere Röhre)
- 14: Schneidkante (innere Röhre)

## Patentansprüche

1. Vorrichtung zum Schneiden und Absaugen von Gewebe aus dem menschlichen oder tierischen Körper, vorzugsweise zum Einsatz in der Ophthalmologie, insbesondere zur Vitrektomie, zum Netzhautpeeling etc., mit einer äußeren Röhre (1) und einer in der äußeren Röhre (1) konzentrisch mit geringem Spiel alternierend verschiebbaren inneren Röhre (11), wobei die äußere Röhre (1) am freien Ende (2) geschlossen ist und nahe dem freien Ende (2) eine erste und eine zweite seitliche Öffnung (3, 4) mit jeweils zumindest einer inneren Schneidkante (7, 8) aufweist, wobei die innere Röhre (11) am freien Ende offen ist und dort eine äußere Schneidkante (12) aufweist, wobei die innere Röhre (11) nahe dem freien Ende mindestens eine seitliche Öffnung (13) mit mindestens einer weiteren äußeren Schneidkante (14) aufweist und wobei die Schneidkanten (7, 8, 12, 14) der äußeren Röhre (11) und der inneren Röhre (1) beim Verschieben der inneren Röhre (11) schneidend zusammen wirken,
**dadurch gekennzeichnet, dass** die erste und die zweite Öffnung (3, 4) jeweils als asymmetrischer Kreis, als asymmetrische Ellipse oder als asymmetrisches Oval ausgebildet sind und dass durch den asymmetrischen Bereich (5) der ersten und zweiten Öffnung (3, 4) an der äußeren Röhre (1) ein dünner Steg (6) ausgebildet ist.

2. Vorrichtung nach Anspruch 1, **dadurch gekennzeichnet, dass** der Steg (6) zumindest abschnittsweise orthogonal zur Längsachse der äußeren Röhre (1) verläuft.

3. Vorrichtung nach Anspruch 1 oder 2, **dadurch gekennzeichnet, dass** die erste und die zweite Öffnung (3, 4) der äußeren Röhre (1) achsensymmetrisch zueinander ausgebildet sind, wobei der Steg (6) zumindest abschnittsweise einen Teil einer Symmetrieachse bildet.

4. Vorrichtung nach einem der Ansprüche 1 bis 3, **dadurch gekennzeichnet, dass** an der ersten und der zweiten Öffnung (3, 4) jeweils eine erste Schneidkante (7) und eine zweite Schneidkante (8) ausgebildet ist.

5. Vorrichtung nach Anspruch 4, **dadurch gekennzeichnet, dass** die erste Schneidkante (7) in dem kreisförmigen, elliptischen oder ovalen Bereich der ersten und der zweiten Öffnung (3, 4) ausgebildet ist und/oder dass die zweite Schneidkante (8) in dem asymmetrischen Bereich (5) der ersten und der zweiten Öffnung (3, 4) ausgebildet ist.

6. Vorrichtung nach einem der Ansprüche 1 bis 5, **dadurch gekennzeichnet, dass** sich von den Schneidkanten (7, 8) aus, durch die Materialstärke der äußeren Röhre (1) definierte, Schneidflächen (9, 10) nach außen erstrecken.

7. Vorrichtung nach Anspruch 6, **dadurch gekennzeichnet, dass** sich die Schneidfläche/Schneidflächen (9, 10) orthogonal und/oder unter einem Winkel zu der Längsachse der äußeren Röhre (1) erstreckt/erstrecken.

8. Vorrichtung Anspruch 6 oder 7, **dadurch gekennzeichnet, dass** die Schneidfläche/Schneidflächen (9, 10) linear und/oder konkav ausgebildet sind.

9. Vorrichtung nach einem der Ansprüche 6 bis 8, **dadurch gekennzeichnet, dass** eine von der ersten Schneidkante (7) nach außen verlaufende erste Schneidfläche (9) der ersten Öffnung (3) und eine von der ersten Schneidkante (7) nach außen verlaufende erste Schneidfläche (9) der zweiten Öffnung (4) V-förmig zueinander angeordnet sind.

10. Vorrichtung nach einem der Ansprüche 1 bis 9, **dadurch gekennzeichnet, dass** die innere Röhre (11) nahe dem freien Ende zwei benachbarte seitliche Öffnungen aufweist, die insgesamt mit drei oder vier äußeren Schneidkanten ausgestattet sind.

11. Vorrichtung nach einem der Ansprüche 1 bis 10, **dadurch gekennzeichnet, dass** die äußere Röhre (1) eine dritte und eine vierte seitliche Öffnung aufweist, die analog zur ersten und zweiten Öffnung (3, 4) ausgebildet sind.

## Claims

1. Device for cutting and drawing off tissue from the human or animal body, preferably for use in ophthalmology, in particular for vitrectomy, for retina peeling, etcetera, having an outer tube (1) and an inner tube (11) which can be displaced in an alternating manner in the outer tube (1) concentrically with little play, wherein the outer tube (1) is closed at the free end (2) and has close to the free end (2) a first and a second lateral opening (3, 4) each having at least one inner cutting edge (7, 8), wherein the inner tube (11) is open at the free end and at that location has an outer cutting edge (12), wherein the inner tube (11) close to the free end has at least one lateral opening (13) having at least one additional outer cutting edge (14) and wherein the cutting edges (7, 8, 12, 14) of the outer tube (11) and the inner tube (1) cooperate in a cutting manner when the inner tube (11) is displaced,
**characterised in that** the first and the second opening (3, 4) are constructed in each case as an asymmetrical circle, as an asymmetrical ellipse or as an asymmetrical oval and **in that** through the asymmetrical region (5) of the first and the second opening (3, 4) in the outer tube (1) a thin web (6) is formed.

2. Device according to claim 1, **characterised in that** the web (6) extends at least partially in an orthogonal manner with respect to the longitudinal axis of the outer tube (1).

3. Device according to claim 1 or 2, **characterised in that** the first and the second opening (3, 4) of the outer tube (1) are constructed in an axially symmetrical manner with respect to each other, wherein the web (6) at least partially forms a portion of an axis of symmetry.

4. Device according to any one of claims 1 to 3, **characterised in that** at the first and the second opening (3, 4) a first cutting edge (7) and a second cutting edge (8) are formed, respectively.

5. Device according to claim 4, **characterised in that** the first cutting edge (7) is formed in the circular, elliptical or oval region of the first and the second opening (3, 4) and/or **in that** the second cutting edge (8) is formed in the asymmetrical region (5) of the first and the second opening (3, 4).

6. Device according to any one of claims 1 to 5, **characterised in that** cutting faces (9, 10) which are defined by the material thickness of the outer tube (1) extend outwards from the cutting edges (7, 8).

7. Device according to claim 6, **characterised in that** the cutting face/cutting faces (9, 10) extend(s) orthogonally and/or at an angle relative to the longitudinal axis of the outer tube (1).

8. Device according to claim 6 or 7, **characterised in that** the cutting face/cutting faces (9, 10) is/are constructed in a linear and/or concave manner.

9. Device according to any one of claims 6 to 8, **characterised in that** a first cutting face (9) of the first opening (3), which face extends outwards from the first cutting edge (7), and a first cutting face (9) of the second opening (4), which face extends outwards from the first cutting edge (7), are arranged in a V-shaped manner with respect to each other.

10. Device according to any one of claims 1 to 9, **characterised in that** the inner tube (11) close to the free end has two adjacent lateral openings which are provided in total with three or four outer cutting edges.

11. Device according to any one of claims 1 to 10, **characterised in that** the outer tube (1) has a third and a fourth lateral opening, which are constructed in a similar manner to the first and second openings (3, 4).

## Revendications

1. Dispositif de coupe et d'aspiration de tissu du corps humain ou animal, de préférence en vue d'une utilisation en ophtalmologie, en particulier en vitrectomie, pour le peeling de la rétine, etc., avec un tube extérieur (1) et une tube intérieur (11) pouvant coulisser de façon concentrique dans le tube extérieur (1) de façon alternée avec un faible jeu, le tube extérieur (1) étant fermé à l'extrémité libre (2) et présentant près de l'extrémité libre (2) un premier et un deuxième orifice latéral (3, 4) avec respectivement au moins une arête de coupe (7, 8) intérieure, le tube intérieur (11) étant ouvert à l'extrémité libre et présentant à cet endroit une arête de coupe (12) extérieure, le tube intérieur (11) présentant près de l'extrémité libre au moins un orifice (13) latéral avec au moins une autre arête de coupe (14) extérieure, et les arêtes de coupe (7, 8, 12, 14) du tube extérieur (11) et du tube intérieur (1) coopérant dans une action de coupe lors du coulissement du tube intérieur (11),
**caractérisé en ce que** le premier et le deuxième orifice (3, 4) sont constitués respectivement en tant que cercle asymétrique, en tant qu'ellipse asymétrique ou en tant qu'ovale asymétrique, et **en ce qu'**une nervure (6) mince est constitué par la zone (5) asymétrique du premier et du deuxième orifice (3, 4) sur le tube extérieur (1).

2. Dispositif selon la revendication 1, **caractérisé en ce que** la nervure (6) s'étend au moins par tronçons à angle droit par rapport à l'axe longitudinal du tube extérieur (1).

3. Dispositif selon la revendication 1 ou 2, **caractérisé en ce que** le premier et le deuxième orifice (3, 4) du tube extérieur (1) sont constitués avec une symétrie axiale l'un par rapport à l'autre, la nervure (6) formant au moins par tronçons une partie de l'axe de symétrie.

4. Dispositif selon l'une des revendications 1 à 3, **caractérisé en ce que** respectivement une première arête de coupe (7) et une deuxième arête de coupe (8) sont constituées sur le premier et le deuxième orifice (3, 4).

5. Dispositif selon la revendication 4, **caractérisé en ce que** la première arête de coupe (7) est constituée dans la zone circulaire, elliptique ou ovale du premier et du deuxième orifice (3, 4), et/ou **en ce que** la deuxième arête de coupe (8) est constituée dans la zone asymétrique (5) du premier et du deuxième orifice (3, 4).

6. Dispositif selon l'une des revendications 1 à 5, **caractérisé en ce que** des surface de coupe (9, 10) définies par l'épaisseur de matériau du tube extérieur (1) s'étendent vers l'extérieur à partir des arêtes de coupe (7, 8).

7. Dispositif selon la revendication 6, **caractérisé en ce que** la surface de coupe/les surfaces de coupe (9, 10) s'étend/s'étendent à angle droit et/ou en formant un angle par rapport à l'axe longitudinal du tube extérieur (1).

8. Dispositif selon la revendication 6 ou 7, **caractérisé en ce que** la surface de coupe/les surfaces de coupe (9, 10) sont constituées de façon linéaire et/ou concave.

9. Dispositif selon l'une des revendications 6 à 8, **caractérisé en ce qu'**une première surface de coupe (9) du premier orifice (3) s'étendant vers l'extérieur à partir de la première arête de coupe (7) et une première surface de coupe (9) du deuxième orifice (4) s'étendant vers l'extérieur à partir de la première arête de coupe (7) sont disposées en forme de V l'une par rapport à l'autre.

10. Dispositif selon l'une des revendications 1 à 9, **caractérisé en ce que** le tube intérieur (11) présente près de l'extrémité libre deux orifices latéraux voisins qui sont équipés au total de trois ou quatre arêtes de coupe extérieures.

11. Dispositif selon l'une des revendications 1 à 10, **caractérisé en ce que** le tube extérieur (1) présente un troisième et un quatrième orifice latéral qui sont constitués de façon analogue au premier et au deuxième orifice latéral (3, 4).
